# EUROPEAN PATENT SPECIFICATION

(11) **EP 0 560 000 B1**
(45) Date of publication and mention of the grant of the patent: **23.09.1998**
(21) Application number: 92830593.7
(22) Date of filing: 30.10.1992
(51) Int. Cl.: A61M 1/10

(54) **Ventricular pumping device**
Ventrikulare Pumpenvorrichtung
Dispositif de pompage ventriculaire

(30) Priority: 05.11.1991 IT MI912926
(43) Date of publication of application: 15.09.1993
(73) Proprietor: Parravicini, Roberto, I-41100 Modena (IT)
(72) Inventor: Parravicini, Roberto, I-41100 Modena (IT)
(74) Representative: Cicogna, Franco

(56) References cited:
- EP-A- 0 480 102
- GB-A- 2 239 675
- US-A- 4 817 586
- US-A- 4 944 722
- US-A- 4 957 504

## Description

### BACKGROUND OF THE INVENTION

The present invention relates to a ventricular pumping device which can be operated from the outside.

As is known, patients affected by cardiac insufficiency are at present subjected to surgical operations for implanting aiding systems adapted to promote the pumping performance by the heart.

On the other hand, prior apparatus are not sufficient in those cases in which the patient heart is not able of providing a satisfactory pumping effect.

Moreover, prior aiding systems of the above mentioned type have a small application range, because of blood coagulation problems and because they involve a deterioration of the host organism.

The document US-A-4 944 722 discloses a cable-driven percutaneous insertable intravenous axial flow blood pump preloadable to provide a substantially constant purge pressure unaffected by manipulation of the pump drive cable or cable sheath.

The document US-A-4 817 586 discloses an intravascular axial flow blood pump the outside diameter of which is reduced without reducing the size of its journal bearings by causing the pumped blood stream to exit through apertures in the cylindrical outside wall of the pump housing between the rotor blades and the rotor journal, thereby allowing the journal to have a diameter of the housing without the need for a space-consuming blood flow path around the journal.

The document US-A-4 957 504, on which the preamble of the main claim has been based, discloses a blood pump the impeller of which constitutes the rotor of a driving electric motor and a cylindric body of which constitutes the stator of said electric motor.

Thus, the electric motor of this prior document is a very complex electric motor, of specifically designed construction, which requires a lot of driving magnets as well as a very complex electric control device.

Furthermore, this electric motor does not include any branching duct like that taught by the Applicant and is mainly provided for coupling to the aorta, for example, by a parallel arrangement of two pumping units which, in addition to greatly complicating the device construction, could involve proper operation problems.

### SUMMARY OF THE INVENTION

Accordingly, the aim of the present invention is to overcome the above mentioned drawbacks, by providing a ventricular pumping device, to be operated from outside, which is specifically designed for directly providing the pumping action, in order to promote the blood flow and to hold a proper blood flow rate.

Within the scope of the above mentioned aim, a main object of the present invention is to provide such a ventricular pumping device which can be supplied from outside, without requiring any further surgical operations for repairing possible malfunctioning components thereof.

Yet another object of the present invention is to provide such a ventricular pumping device which is very reliable in operation, is very simple from a mere constructional standpoint and does not require maintenance operations.

According to one aspect of the present invention, the above mentioned aim and objects, as well as yet other objects, which will become more apparent hereinafter, are achieved by a ventricular pumping device, according to Claim 1.

### BRIEF DESCRIPTION OF THE DRAWINGS

Further characteristics and advantages of the invention will become more apparent hereinafter from the following detailed disclosure of a preferred, though not exclusive, embodiment thereof which is illustrated, by way of an indicative, but not limitative example in the accompanying drawing, where:

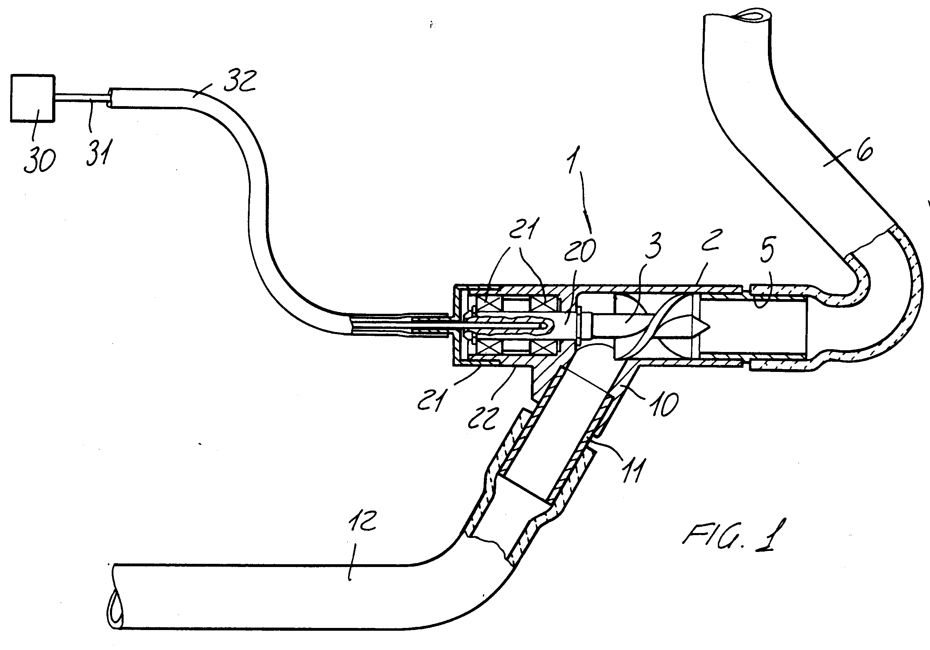

Figure 1 is a cross-sectional view of the ventricular pumping device according to the present invention.

### DESCRIPTION OF THE PREFERRED EMBODIMENT

With reference to the figure of the accompanying drawing, the ventricular pumping device adapted to be operated from outside, according to the invention, which has been generally indicated at the reference number 1, comprises a cylindric body 2 in the inside of which there is housed an impeller 3, having advantageously a propeller configuration and being coaxially mounted on the cylindric body 2.

At an axial end of the body 2 there is provided an inlet 5 to which an inlet channel 6 is connected, said channel being adapted to be communicated with the right auricle or the left auricle of a patient heart.

If desired, it is also possible to connect the two auricles and, in this case, two discrete pumping devices will be used.

Downstream, with respect to the impeller 3, of the cylindric body 2, there extends a branching leg 10, to which there is connected an outlet 11 communicating with an outlet channel 12 which can be respectively connected either to the pulmonary artery or to the aorta.

As shown, the impeller 3 is coupled to a small shaft 20 which projects, in a tight way, from the impeller chamber and being supported, by means of bearings 21, in an end-piece 22, arranged at the opposite side of the inlet 5.

The impeller 3 is driven by means of driving means, indicated at the reference number 30, which are arranged outside of the chest of the patient, whereas the pumping device will be arranged, by a surgical operation, inside the patient chest.

As shown, the driving means 30 are coupled, by means of a flexible cable 31, which is encompassed by a tightness protecting sheath 32, to the shaft 20 so as to cause the impeller to turn.

The motor 30 is preferable a small electric motor the speed of which can be adjusted so as to provide the desired blood flow rate.

Thus, in the case of a cardiac insufficiency, the pumping device according to the invention will facilitate the operation of the heart auricles so as to promote the blood flow and hold a proper blood flow rate.

From the above disclosure it should be apparent that the invention fully achieves the intended aim and objects.

In particular, it is to be pointed out that the subject ventricular pumping device comprises driving means which can be arranged outside of the chest of the patient and, accordingly, do not require for their maintenance and power supply any surgical operations.

The invention as disclosed is susceptible to several modifications and variations.

Moreover, all of the details can be replaced by other technically equivalent elements.

In practicing the invention, the used materials, as well as the contingent size and shapes can be any according to requirements.

## Claims

1. A ventricular pumping device (1) implantable in a thorax of a patient requiring ventricular assistance, said device comprising a cylindric body (2) housing an impeller (3) driven by an adjustable speed electric motor (30) therein, said cylindric body having an inlet and an outlet, said inlet (5) of said cylindric body being arranged coaxially with respect to said cylindric body and being provided for coupling to an inlet channel (6) provided for communicating with a myocardic auricle, characterized in that said cylindric body comprises moreover an offset branching leg (10) extending at an angle from said cylindric body and being provided for connection to an outlet channel (12) provided for communicating with a pulmonary artery or the aorta, that said branching leg is arranged downstream of said impeller (3) and is coupled to an outlet (11) communicating with said outlet channel (12) and that said adjustable speed electric motor (30) is moreover provided to be arranged outside said patient thorax for driving said impeller, so as to provide for an adjustable flow-rate blood flow.

2. A ventricular pumping device, according to Claim 1, characterized in that said impeller (3) has a propeller configuration.

3. A ventricular pumping device, according to Claim 1, characterized in that said impeller (3) is arranged coaxially of said cylindric body (2).

4. A ventricular pumping device, according to Claim 1, characterized in that said impeller is supported by a shaft (20) which is fluid-tightly rotatably mounted through bearings (21) on an end piece (22) arranged on a side opposite to said inlet (5).

5. A ventricular pumping device, according to Claim 1, characterized in that adjustable speed electric motor (30) is connected to said shaft (20) by a flexible cable (31) encompassed by a protecting sheath (32).

## Patentansprüche

1. Eine ventrikuläre Pumpenvorrichtung (1) zur Implantation in den Thorax eines Patienten, der ventrikuläre Unterstützung benötigt, wobei dieses Gerät einen zylindrischen Körper (2) umfaßt, der ein von einem Elektromotor (30) mit regelbarer Drehzahl angetriebenes Laufrad (3) umschließt, wobei dieser zylindrische Körper einen Einlaß und einen Auslaß besitzt und dieser Einlaß (5) dieses zylindrischen Körpers koaxial zu diesem zylindrischen Körper angeordnet und zum Anschluß an einen Einlaßkanal (6) angepaßt ist, der für die Verbindung mit dem Myokard eines Vorhofs bereitgestellt ist, dadurch gekennzeichnet, daß dieser zylindrische Körper ferner eine versetzte Verzweigung (10) umfaßt, die einen Winkel mit diesem zylindrischen Körper bildet und für die Verbindung an einen Auslaßkanal (12) bereitgestellt ist, der für die Kommunikation mit einer Lungenarterie oder der Aorta vorgesehen ist, daß diese Verzweigung in Fließrichtung unterhalb dieses Laufrades (3) angeordnet und mit einem Auslaß (11) verbunden ist, der an diesem Auslaßkanal (12) angeschlossen ist, und daß ferner dieser Elektromotor (30) mit regelbarer Drehzahl dafür vorgesehen ist, zum Antreiben dieses Laufrades außerhalb dieses Patiententhoraxes angebracht zu werden, um so einen Blutfluß mit einstellbarem Durchsatz bereitzustellen.

2. Eine ventrikuläre Pumpenvorrichtung nach Anspruch 1, dadurch gekennzeichnet, daß dieses Laufrad (3) wie ein Propeller konfiguriert ist.

3. Eine ventrikuläre Pumpenvorrichtung nach Anspruch 1, dadurch gekennzeichnet, daß dieses Laufrad (3) koaxial zu diesem zylindrischen Körper (2) angeordnet ist.

4. Eine ventrikuläre Pumpenvorrichtung nach Anspruch 1, dadurch gekennzeichnet, daß dieses Laufrad von einer Welle (20) getragen wird, die fluiddicht und auf Lagern (21) drehbar auf einem Endstück (22) angebracht ist, das an einem diesem Einlaß (5) gegenüberliegenden Ende angebracht ist.

5. Eine ventrikuläre Pumpenvorrichtung nach Anspruch 1, dadurch gekennzeichnet, daß dieser Elektromotor (30) mit regelbarer Drehzahl an dieser Welle (20) über ein flexibles Kabel (31) angeschlossen ist, das von einer Schutzhülle (32) umgeben ist.

## Revendications

1. Dispositif de pompage ventriculaire (1) pouvant être implanté dans le thorax d'un patient nécessitant une assistance ventriculaire, ledit dispositif comportant un corps cylindrique (2) contenant un rotor (3) avec aubes entraîné par un moteur électrique à vitesse réglable (30) à l'intérieur de celui-ci, ledit corps cylindrique ayant une entrée et une sortie, ladite entrée (5) dudit corps cylindrique étant aménagée de manière coaxiale par rapport audit corps cylindrique et étant prévue pour être couplé à un canal d'entrée (6) prévu pour communiquer avec une oreillette myocardique, caractérisé en ce que ledit corps cylindrique comporte par ailleurs une branche déviée (10) qui s'étend à un angle dudit corps cylindrique et étant prévue pour être raccordée à un canal de sortie (12) prévu pour communiquer avec l'artère pulmonaire ou l'aorte, en ce que ladite branche est placée en aval de dudit rotor (3) et qu'elle est couplée à une sortie (11) communiquant avec ledit canal de sortie (12) et en ce que ledit moteur électrique à vitesse réglable (30) est par ailleurs prévu pour être aménagé à l'extérieur du thorax du patient pour entraîner ledit rotor, de manière à fournir un flux sanguin à débit réglable.

2. Dispositif de pompage ventriculaire selon la revendication 1, caractérisé en ce que ledit rotor (3) a une configuration d'hélice.

3. Dispositif de pompage ventriculaire selon la revendication 1, caractérisé en ce que ledit rotor (3) est aménagé de manière coaxiale par rapport audit corps cylindrique (2).

4. Dispositif de pompage ventriculaire selon la revendication 1, caractérisé en ce que ledit rotor est soutenu par un arbre (20) qui est monté de manière étanche et en rotation au moyen de coussinets (21) sur une extrémité (22) ménagée d'un côté en face de ladite entrée (5).

5. Dispositif de pompage ventriculaire selon la revendication 1, caractérisé en ce que ledit moteur électrique à vitesse réglable (30) est raccordé audit arbre (20) au moyen d'un câble souple (31) dans une enveloppe protectrice (32).
